# NOUVEAU FASCICULE DE BREVET EUROPEEN

(11) **EP 1 768 789 B3**
(45) Date de publication du présent fascicule: **15.08.2012**
(45) Mention de la délivrance du brevet: 02.01.2008
(21) Numéro de dépôt: 05778090.0
(22) Date de dépôt: 16.06.2005
(51) Int. Cl.: B05B 11/00

(54) **DISPOSITIF DE DISTRIBUTION DE PRODUIT FLUIDE.**
SPENDER FÜR EIN FLÜSSIGPRODUKT
FLUID PRODUCT DISPENSING DEVICE

(30) Priorité: 16.06.2004 FR 0406526
(43) Date de publication de la demande: 04.04.2007
(73) Titulaire: Valois SAS, F-27110 Le Neubourg (FR)
(72) Inventeur: MARGHERITIS, Antonio, I-21033 CITTIGLIO (IT); PRUVOT, Samuel, F-27430 SAINT ETIENNE DU VAUVRAY (FR); PETIT, Ludovic, F-27110 VITOT (FR); HERRY, Serge, F-27400 QUATREMARE (FR); FAGOT, Christophe, F-78250 MEZY (FR)
(74) Mandataire: CAPRI
(86) Numéro de dépôt international: PCT/FR2005/050454
(87) Numéro de publication internationale: WO 2006/003343

(56) Documents cités:
- ES-A1- 375 103
- FR-A- 2 475 641
- FR-A- 2 547 364
- US-A- 5 503 306

## Description

La présente invention concerne un dispositif de distribution de produit fluide comme connu du document FR2547364A1.

Les dispositifs de distribution de produit fluide comportent généralement un réservoir contenant le produit fluide (liquide, pâteux ou pulvérulent) à distribuer, et un organe de distribution monté sur ledit réservoir, tel qu'une pompe ou une valve, pour distribuer sélectivement le contenu dudit réservoir. Une tête de distribution est assemblée sur l'organe de distribution pour actionner celui-ci, afin de distribuer manuellement une dose de produit à chaque actionnement. Lorsque l'organe de distribution est une pompe, cette pompe comporte généralement un corps de pompe définissant une chambre de pompe dans laquelle coulisse un piston entre une position de repos et une position de distribution. Le piston est déplacé de sa position de repos vers sa position de distribution par une pression exercée par l'utilisateur sur la tête de distribution. La chambre de dosage peut comporter un clapet d'entrée et un clapet de sortie, et le dispositif peut comporter un obturateur au niveau de son orifice de distribution, généralement prévu dans la tête de distribution. De manière classique, le corps de pompe est généralement fixé sur le réservoir, d'une manière quelconque connue, par exemple au moyen d'une bague de fixation. Le piston est alors monté coulissant à l'intérieur dudit corps de pompe et est déplacé par ladite tête de distribution. Cette mise en oeuvre présente un certains nombres d'inconvénients. Ainsi, la surface de glissement du piston dans le corps de pompe doit être parfaitement lisse et régulière pour éviter un dysfonctionnement de cette pompe, tel qu'une perte d'étanchéité, ou un blocage du piston. Ceci impose des précautions très importantes lors de l'assemblage du corps de pompe sur le réservoir, pour éviter que ce corps de pompe ne soit déformé lors de cet assemblage. En particulier, un certain nombre de modes d'assemblage sont très difficiles à réaliser lorsque l'on souhaite fixer un corps de pompe sur un col de réservoir. Ceci est particulièrement vrai lorsqu'il s'agit d'un encliquetage à l'intérieur du col, puisque si ce col est rigide, il est généralement nécessaire de pouvoir déformer la partie encliquetée, à savoir le corps de pompe, ce qui génère un risque de déformation de la surface de glissement du piston avec les inconvénients susmentionnés.

Un autre inconvénient des pompes classiques est que la chambre de dosage est généralement disposée relativement en éloignement de l'orifice de distribution, et il est alors nécessaire de prévoir à la fois un clapet de sortie de la chambre de dosage pour assurer une définition précise de ce dosage, et un obturateur au niveau de l'orifice de distribution pour éviter tout risque de contamination du produit entre deux actionnements. De plus, ceci implique généralement l'utilisation de deux ressorts, un pour ramener le piston vers sa position de repos, et l'autre pour solliciter l'obturateur vers sa position de fermeture.

Par ailleurs, un autre inconvénient des pompes existantes est lié à l'assemblage qui prévoit de fixer une pompe (ou certains éléments de la pompe) à un réservoir au moyen d'une bague de fixation ou similaire. Lorsque la partie de pompe à fixer est le corps de pompe, l'assemblage de celui-ci sur le réservoir au moyen d'une bague de fixation ne pose pas de problème majeur, mais nécessite quand même deux étapes distinctes d'assemblage (pré-assemblage du corps de pompe dans la bague ou sur le réservoir, puis fixation de la bague). Lorsque l'élément de pompe à assembler sur le réservoir est une pièce interne de la pompe, cet assemblage peut en outre s'avérer plus difficile et risque d'endommager des parties d'étanchéité dudit élément. II s'en suit une pompe généralement complexe à fabriquer et à assembler, ce qui peut s'avérer néfaste du point de vue des performances de la pompe et de son coût.

La présente invention a pour but de fournir un dispositif de distribution de produit fluide qui ne reproduit pas les inconvénients susmentionnés.

Plus particulièrement, la présente invention a pour but de fournir un tel dispositif de distribution de produit fluide qui élimine tout risque d'altération de la surface de glissement du piston lors de l'assemblage du dispositif.

La présente invention a aussi pour but de fournir un tel dispositif de distribution de produit fluide qui comporte un minimum de pièces constitutives. En particulier, l'invention élimine une, respectivement deux pièce(s) d'assemblage en réalisant le piston monobloc avec les moyens de fixation, respectivement le réservoir. De plus, l'invention élimine le besoin de cumuler un clapet de sortie de la chambre de dosage et un obturateur de l'orifice de distribution, et permet de réaliser une pompe avec obturateur ne comportant qu'un seul ressort.

La présente invention a aussi pour but de fournir un tel dispositif de distribution de produit fluide qui soit simple et peu coûteux à fabriquer et à assembler, de fonctionnement sûr et fiable, et qui évite tout risque de contamination du produit contenu dans ledit dispositif.

La présente invention a donc pour objet un dispositif de distribution de produit fluide, comportant un réservoir, une tête de distribution incorporant un orifice de distribution, une chambre de dosage et un piston coulissant dans ladite chambre de dosage entre une position de repos et une position de distribution, ledit piston étant monté fixement sur ledit réservoir, ledit piston étant réalisé de manière monobloc avec ledit réservoir et/ou avec des moyens de fixation adaptés à se fixer sur ledit réservoir.

Avantageusement, ledit piston est fixé sur le réservoir par des moyens de fixation, lesdits moyens de fixation étant reliés de manière monobloc audit piston.

Avantageusement, lesdits moyens de fixation sont des moyens d'encliquetage, d'emmanchement ou de vissage.

Selon une première variante de réalisation, ledit piston est fixé à l'extérieur d'un col dudit réservoir.

Selon une seconde variante de réalisation, ledit piston est fixé à l'intérieur d'un col dudit réservoir.

Selon une troisième variante de réalisation, ledit piston est fixé à l'extérieur et à l'intérieur d'un col dudit réservoir.

Selon une quatrième variante de réalisation, ledit piston est réalisé de manière monobloc avec ledit réservoir.

Avantageusement, ledit piston coulisse dans un corps de pompe disposé dans la tête de distribution.

Avantageusement, ledit corps de pompe est solidaire d'un obturateur coulissant dans ladite tête de distribution entre une position de fermeture et une position d'ouverture de l'orifice de distribution.

Avantageusement, ledit obturateur comporte un second piston coulissant dans un cylindre de ladite tête de distribution.

Avantageusement, ledit piston comporte un clapet d'entrée de la chambre de pompe.

Avantageusement, ledit piston est fixé sur le réservoir avec interposition d'un joint entre le piston et le réservoir.

Avantageusement, ledit joint est déformée lors de la fixation dudit piston sur ledit réservoir.

Avantageusement, le piston comporte des moyens de fixation coopérant avec l'extérieur d'un col du réservoir, ledit joint coopérant avec l'intérieur dudit col.

En variante, le piston comporte des moyens de fixation coopérant avec l'intérieur d'un col du réservoir.

Avantageusement, lesdits moyens de fixation comprennent ledit joint qui se déforme entre le piston et l'intérieur du col du réservoir pour fixer le piston par emmanchement étanche dans ledit col.

Avantageusement, l'une parmi la surface intérieure du col du réservoir et la surface extérieure correspondante du piston comporte un profil d'étanchéité coopérant avec ledit joint à l'état monté, de préférence avec un profil d'étanchéité complémentaire dudit joint.

Avantageusement, ledit joint comporte une projection qui, lors de l'emmanchement du piston, est déformée sous le col du réservoir, à l'intérieur de celui-ci, pour fixer le piston de manière indémontable.

Avantageusement, ledit piston comporte des moyens d'encliquetage coopérant avec la surface intérieure et/ou la surface extérieure d'un col dudit réservoir.

Avantageusement, lesdits moyens d'encliquetage comportent un profil d'encliquetage adapté à s'encliqueter dans, respectivement autour dudit col.

Avantageusement, ledit col comporte un profil d'encliquetage complémentaire coopérant avec ledit profil d'encliquetage dudit piston.

Avantageusement, le piston comporte un filetage coopérant avec un filetage correspondant prévu à l'intérieur ou à l'extérieur d'un col dudit réservoir.

Avantageusement, au moins un desdits filetages comporte des moyens empêchant le dévissage, tel qu'un filet interrompu.

Avantageusement, ledit piston comporte des premiers moyens d'encliquetage coopérant avec l'intérieur d'un col du réservoir et des seconds moyens d'encliquetage coopérant avec l'extérieur dudit col.

Avantageusement, l'intérieur et/ou l'extérieur dudit col comporte un profil d'encliquetage complémentaire.

Avantageusement, le bord supérieur d'un col du réservoir comporte deux branches axiales coopérant chacune avec ledit piston, de préférence avec interposition d'un joint.

Avantageusement, lesdits moyens d'encliquetage sont formés directement sur le piston.

Avantageusement, une douille est interposée entre le piston et un col dudit réservoir.

Avantageusement, lesdits moyens d'encliquetage sont formés sur ladite douille.

Avantageusement, un joint est interposé entre ladite douille et le col du réservoir.

Avantageusement, ledit piston est directement emmanché à force à l'intérieur d'un col dudit réservoir.

D'autres caractéristiques et avantages de la présente invention apparaîtront plus clairement au cours de la description détaillée suivante de plusieurs modes de réalisation de celle-ci, faite en référence aux dessins joints, donnés à titre d'exemples non limitatifs, et sur lesquels :
- les figures 1 et 2 sont des vues schématiques en section transversale d'un dispositif de distribution de produit fluide selon un mode de réalisation avantageux de la présente invention, respectivement avant et après assemblage ;
- les figures 3a et 3b sont des vues de détails d'une variante de réalisation des moyens de fixation, respectivement avant et après assemblage ;
- la figure 4 est une vue similaire à la figure 3b, montrant une autre variante de réalisation des moyens de fixation, en position assemblée ;
- la figure 5 est une vue schématique en section transversale partielle d'un dispositif de distribution de produit fluide selon un autre mode de réalisation avantageux de l'invention ;
- la figure 6 est une vue partielle en section transversale d'une autre variante de réalisation d'un dispositif de distribution de produit fluide selon l'invention ;
- la figure 7 est une vue similaire à la figure 5, montrant encore une autre variante de réalisation de la présente invention ;
- la figure 8 est une vue similaire à celles des figures 5 et 7, représentant encore une autre variante de réalisation de la présente invention ;
- les figures 9 à 10 représentent des vues schématiques en section transversale d'un dispositif de distribution de produit fluide réalisé selon encore une autre variante de réalisation de l'invention, respectivement avant et après assemblage ;
- les figures 11a et 11b sont des vues schématiques en section transversale agrandies des détails A et B des figures 9 et 10 ;
- les figures 12a et 12b sont des vues similaires à celles des figures 11a et 11b, montrant une variante de réalisation de la présente invention ;
- les figures 13a et 13b sont des vues similaires aux figures 12a et 12b, représentant une autre variante de réalisation de l'invention ;
- les figures 14 et 15 sont des vues schématiques en section transversale d'un dispositif de distribution de produit fluide selon encore un autre mode de réalisation de la présente invention, respectivement avant et après assemblage ;
- les figures 16 et 17 sont des vues schématiques en section transversale des détails C et D des figures 14 et 15 ;
- les figures 18 et 19 sont des vues similaires aux figures 16 et 17, montrant des variantes de réalisation de l'invention ;
- la figure 20 est une vue schématique en section transversale d'un dispositif de distribution de produit fluide selon encore un autre mode de réalisation de la présente invention ;
- la figure 21 est une vue similaire à celle de la figure 20 montrant également encore un autre mode de réalisation de la présente invention ;
- la figure 22 est une vue schématique en section transversale du détail E de la figure 20 ;
- la figure 23 est une vue similaire à celle de la figure 22 montrant une variante de réalisation de la présente invention ; et
- la figure 24 est une vue schématique partielle en section transversale d'encore une autre variante des moyens de fixation selon la présente invention.

Cette invention concerne un dispositif de distribution de produit fluide comportant un réservoir 10 contenant un produit fluide, par exemple un produit pharmaceutique cosmétique ou du domaine de la parfumerie. Ce réservoir 10 comporte de préférence un col 11 définissant une ouverture permettant l'insertion et la fixation d'un organe de distribution, tel qu'une pompe, comme cela sera décrit ci-après. Une tête de distribution 20 est assemblée de manière mobile par rapport au réservoir 10, cette tête 20 comportant un orifice de distribution 21 à travers lequel le produit est distribué à chaque actionnement. Une chambre de dosage 30 est en outre définie dans laquelle coulisse un piston 40 entre une position de repos et une position de distribution. La chambre de dosage permettant de définir précisément la quantité de produit distribuée à chaque actionnement, le déplacement dudit piston 40 provoquant l'expulsion d'une dose de produit.

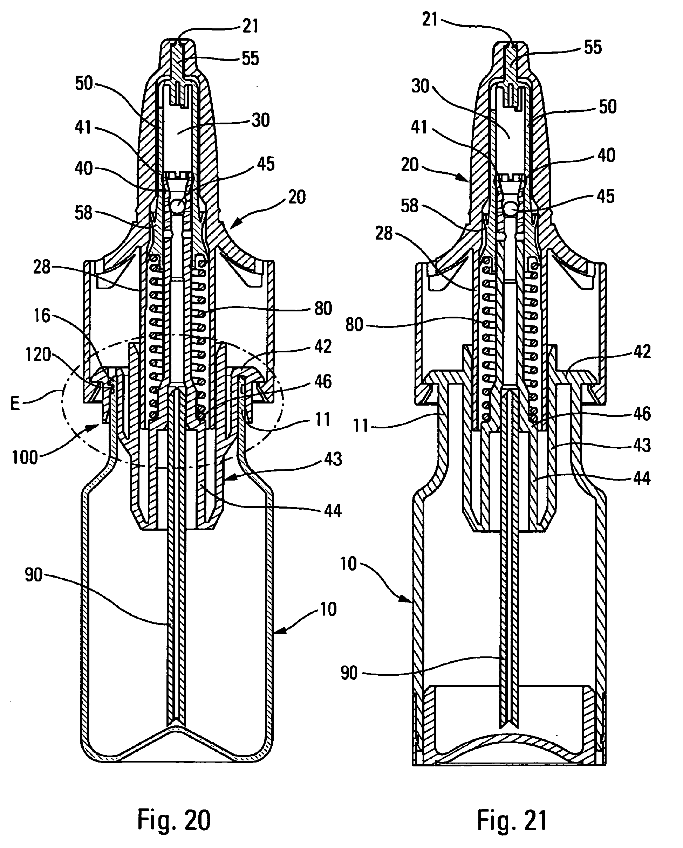

Selon l'invention, ledit piston 40 est monté fixement sur ledit réservoir 10 en étant réalisé de manière monobloc soit directement avec le réservoir, soit avec des moyens de fixation adaptés à se fixer sur ledit réservoir. Il est ainsi possible d'économiser au moins une pièce constitutive par rapport aux assemblages existants. Les différentes figures représentent plusieurs modes et variantes de réalisation de l'invention. Ainsi, sur la figure 21, ledit piston 40 est réalisé de manière monobloc avec ledit réservoir 10, et dans ce cas le produit peut être introduit dans le réservoir par le fond de celui-ci, un bouchon approprié étant ensuite fixé de manière quelconque pour fermer de manière étanche le réservoir. Dans les autres figures, le piston 40 est fixé sur le réservoir par des moyens de fixation 100. Ces différents moyens de fixation seront décrits ci-après en référence aux différentes figures qui représentent différents modes de réalisation.

Les différentes figures représentent un dispositif de distribution de produit fluide particulier, mais il est entendu que la présente invention ne se limite pas aux caractéristiques représentées, notamment en ce qui concerne les parties du dispositif qui ne forment pas les moyens de fixation 100 du piston 40 sur le réservoir 10. Ainsi, la forme de la tête de distribution 20 peut être quelconque et l'orifice 21 n'est pas forcément axial. De même, dans les exemples représentés, le piston 40 coulisse dans un cylindre 50 formant corps de pompe à l'intérieur duquel est définie la chambre de dosage 30. Ce corps de pompe 50 est avantageusement réalisé de manière monobloc avec un obturateur 55 disposé en amont de l'orifice de distribution 21. Par ailleurs, le corps de pompe 50 comporte un second piston 58 adapté à coulisser dans un cylindre 28 de la tête de distribution 20. Un ressort de rappel 80 sollicite ledit obturateur 55 vers sa position de fermeture de l'orifice de distribution 21, et lors de l'actionnement, ledit obturateur peut coulisser vers une position d'ouverture de l'orifice de distribution 21, sous l'effet de la pression du produit, qui déplace ledit second piston 58 dans le cylindre 28 contre la force du ressort de rappel pour ouvrir l'orifice de distribution et permettre l'expulsion de la dose de produit contenue dans la chambre de dosage 30. En effet, pendant le déplacement de l'obturateur 55, le piston 40 coulisse dans le corps 50 pour distribuer la dose. Avantageusement, un clapet d'entrée 45 est prévu en amont de la chambre de dosage 30, et ce clapet d'entrée peut avantageusement être formé sur ledit piston 40. Dans l'exemple représenté, le siège de clapet est formé sur le piston 40, et l'élément de clapet est une bille 45. Bien entendu, tout autre forme de clapet d'entrée est envisageable. Un tube plongeur 90 peut être assemblé dans lie piston 40 pour permettre l'expulsion de la totalité du produit contenue dans le réservoir 10. Avantageusement, le cylindre 28 de la tête de distribution 20 s'étend jusqu'à l'intérieur du col 11 du réservoir 10 lorsque le dispositif est en position d'actionnement ou de distribution, afin d'améliorer la stabilité de l'ensemble et de diminuer les dimensions globales du dispositif. Pour assurer une stabilité et une fixation simple et aisée du piston 40 sur le réservoir 10, le piston 40 comporte avantageusement, à l'extrémité opposée à la partie d'étanchéité 41 formant piston proprement dite et qui coulisse dans le corps de pompe 50, une bride radiale 42 destinée à reposer sur le bord supérieur du col 11 du réservoir 10, ladite bride 42 se prolongeant par un puits axial 43 pénétrant à l'intérieur du col 11 du réservoir 10, et qui se connecte à une partie tubulaire 44 également axiale comportant à son extrémité supérieure (dans le sens représenté sur les figures) ladite partie d'étanchéité 41 formant piston. Cet élément tubulaire 44 comporte avantageusement un épaulement 46 adapté à recevoir l'extrémité du ressort de rappel 80 opposée au second piston 58 du corps de pompe 50. Cet élément tubulaire 44 est creux et relie le tube plongeur 90 au clapet d'entrée 45 et donc à la chambre de dosage 30. Avantageusement, la tête de distribution 20 est en outre encliquetée sur le piston 40 ou sur le col 11 du réservoir 10, ou sur tout élément solidaire de l'un de ces deux éléments, afin d'éviter tout risque de démontage non souhaité ou accidentel de ladite tête 20 par rapport audit réservoir 10. Comme expliqué ci-dessus, ces différentes caractéristiques ne sont représentées qu'à titre d'exemples, et différentes variantes non représentées pourraient également être envisagées.

Selon un premier mode de réalisation de l'invention, représenté sur la figure 21, le piston 40 est donc réalisé de manière monobloc avec le réservoir 10.

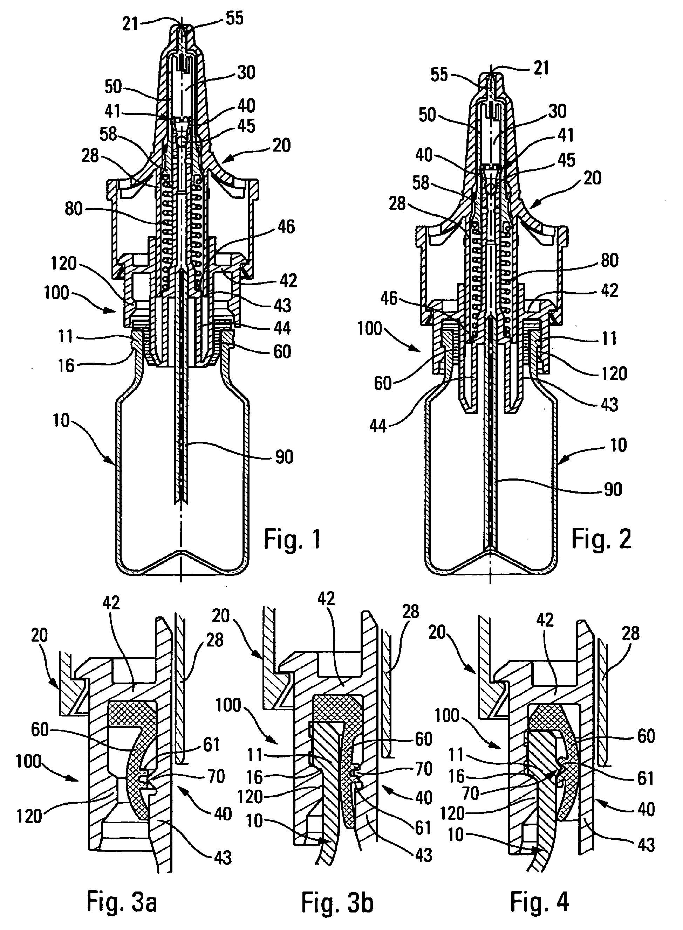

Selon un autre mode de réalisation de l'invention, le piston 40 est fixé à l'extérieur du col 11 du réservoir. Les figures 1 à 4 représentent une telle fixation par encliquetage. A cet effet, des moyens d'encliquetage formant profil d'encliquetage 120 coopérant avec l'extérieur du col 11 sont prévus, de préférence réalisés monoblocs avec le piston 40. Avantageusement, le col 11 peut comporter un profil d'encliquetage complémentaire 16 destiné à coopérer avec le profil d'encliquetage 120 des moyens d'encliquetage. De préférence, un joint 60 est interposé entre le piston 40 et le col 11 du réservoir. De préférence, comme représenté sur les figures 1 à 4, ce joint est déformé lors de la fixation du piston 40 sur le réservoir 10, ce qui favorise une bonne étanchéité à cet endroit.

Les figures 3a et 3b représentent une variante de réalisation du joint 60, qui comporte un profil d'étanchéité 61 adapté à coopérer avec un profil d'étanchéité complémentaire 70 prévu sur la surface extérieure du puits axial 43 du piston 40 qui s'étend à l'intérieur du col 11 du réservoir. Dans l'exemple représenté, ce profil d'encliquetage complémentaire 70 est réalisé sous la forme d'une projection qui coopère par exemple avec deux pattes flexibles 61 du joint 60, qui sont écrasées contre ladite projection lors de la fixation du piston 40 sur le réservoir 10.

La figure 4 représente une variante de réalisation dans laquelle le profil d'encliquetage 70 est réalisé sur la surface intérieure du col 11 du réservoir, auquel cas le joint 60 comporte avantageusement également un profil d'encliquetage complémentaire 61 mais disposé du côté opposé par rapport à la variante représentée en référence aux figures 3a et 3b. Bien entendu, en variante à l'encliquetage, on pourrait aussi imaginer une fixation à l'extérieur du col 11 du réservoir au moyen d'un filetage ou d'un sertissage.

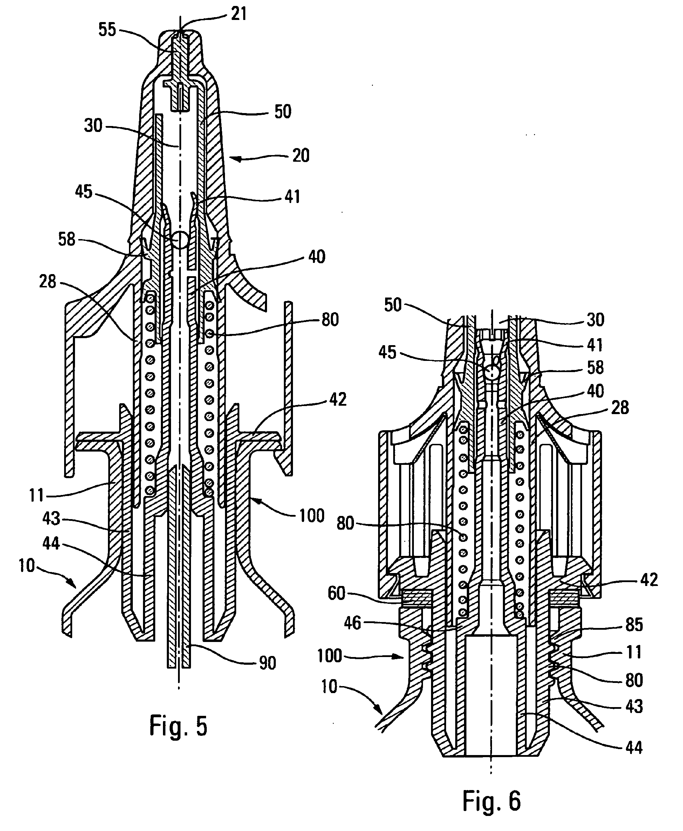

Selon une autre variante de réalisation de l'invention, le piston est fixé à l'intérieur du col 11 du réservoir 10. La figure 5 montre un mode de réalisation dans lequel le puits axial 43 du piston 40 est directement emmanché en force à l'intérieur du col 11 du réservoir. La fixation et l'étanchéité sont obtenues par cet emmanchement à force, qui peut être particulièrement solide puisque la partie emmanchée ne forme plus le corps de pompe dans laquelle doit coulisser le piston de la pompe. Celle-ci peut donc se déformer autant que nécessaire lors de l'emmanchement pour se positionner solidement à l'intérieur du col du réservoir.

La figure 6 montre une variante de réalisation dans laquelle un filetage 80 est prévu sur le piston 40, en particulier sur le puits axial 43, et qui coopère avec un filetage correspondant 85 prévu à l'intérieur du col 11 du réservoir. Avantageusement, un joint 60 est interposé entre le piston 40 et le bord supérieur du réservoir 10, et des moyens empêchant le dévissage peuvent être prévus, par exemple réalisés sous la forme d'un filet interrompu sur l'un ou l'autre desdits filetages 80, 85.

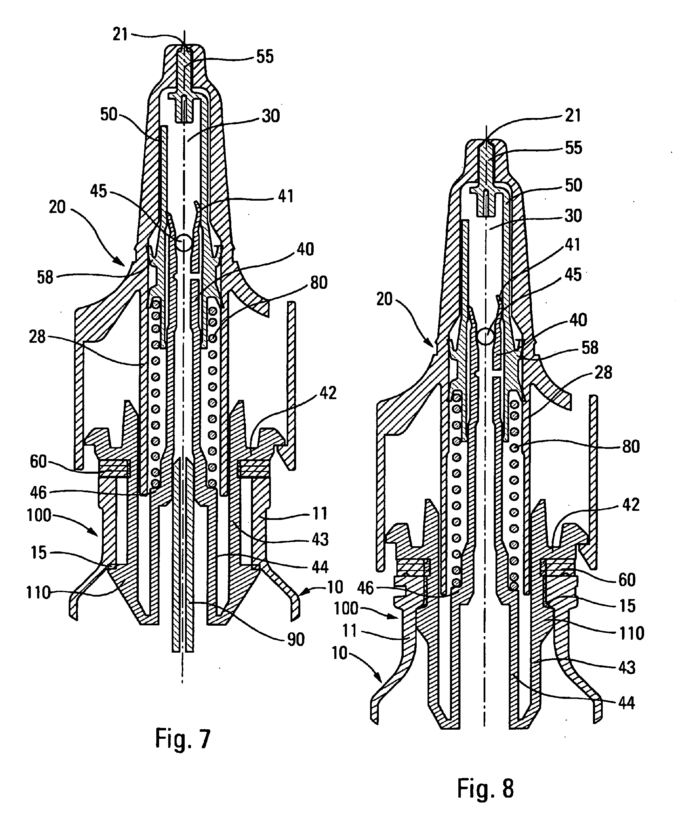

Les figures 7 et 8 représentent deux variantes de réalisation d'un encliquetage à l'intérieur du col 11 du réservoir 10. Dans ces deux cas, le piston 40, notamment le puits axial 43, comporte des moyens d'encliquetage comportant un profil d'encliquetage 110 adapté à coopérer avec le col 11 du réservoir. Avantageusement, un profil d'encliquetage complémentaire 15 peut être prévu à l'intérieur dudit col 11. A nouveau, un tel encliquetage est rendu possible parce que le piston 40, au niveau de sa partie qui se fixe sur le col 11 du réservoir 10, ne définit pas de surface de glissement susceptible d'être déformé de manière irréversible lors de la fixation, et qui risquerait d'altérer le bon fonctionnement du dispositif. Au contraire, la structure représentée et décrite ci-dessus du piston 40 permet une déformation vers l'intérieur de celui-ci, et donc un encliquetage aisé à l'intérieur du col 11 du réservoir. A nouveau, on peut interposer un joint 60 entre le piston 40 et le réservoir 10.

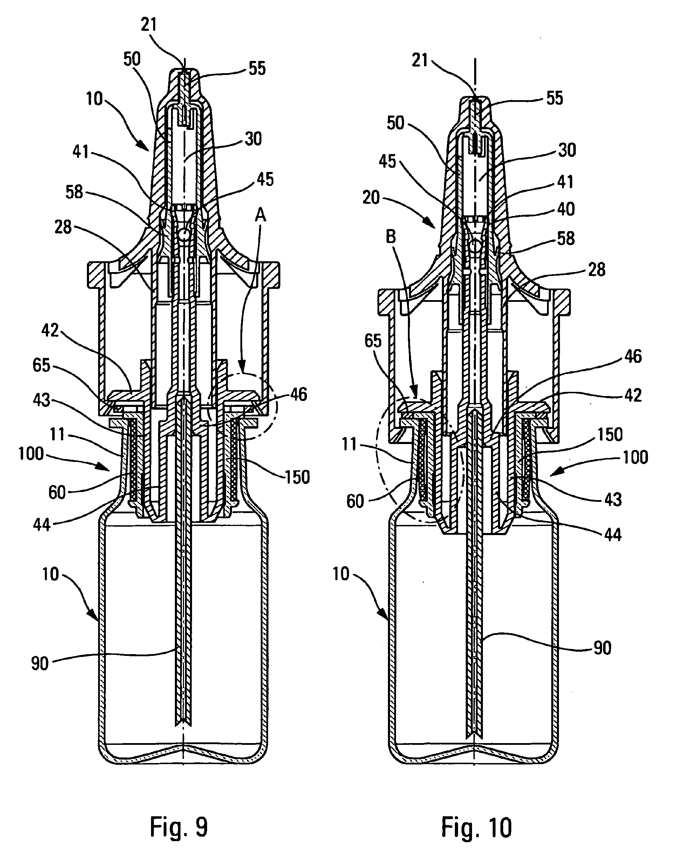

Les figures 9 à 13b représentent d'autres variantes de réalisation. Dans ces exemples, un joint 60 est interposé entre le piston 40 et la surface intérieure du col 11 du réservoir. Les figures 9, 10, 11a et 11b représentent une première variante de réalisation. Dans cette première variante, une douille 150 est également interposée entre le piston 40 et le col 11 du réservoir, cette douille 150 recevant le joint 60. Ce joint 60 peut comporter une extension d'extrémité 65 qui est déformée au tout dernier moment lors de l'assemblage du dispositif, cette déformation provoquant une contrainte du joint 60 et donc une bonne étanchéité. Comme visible sur les figures 9, 10, 11a et 11b, la douille 150 comporte un épaulement supérieur et un épaulement inférieur entre lequel est disposé le joint 60 à l'état non contraint avant assemblage. Lors de la phase finale de l'assemblage, l'extension supérieure 65 du joint 60 est déformée, en l'occurrence déplacée axialement vers le bas, ce qui a pour effet une expansion du joint 60 sur la surface interne du col 11 du réservoir. Cette expansion radiale assure à la fois l'étanchéité et la fixation du piston 40 dans le réservoir 10.

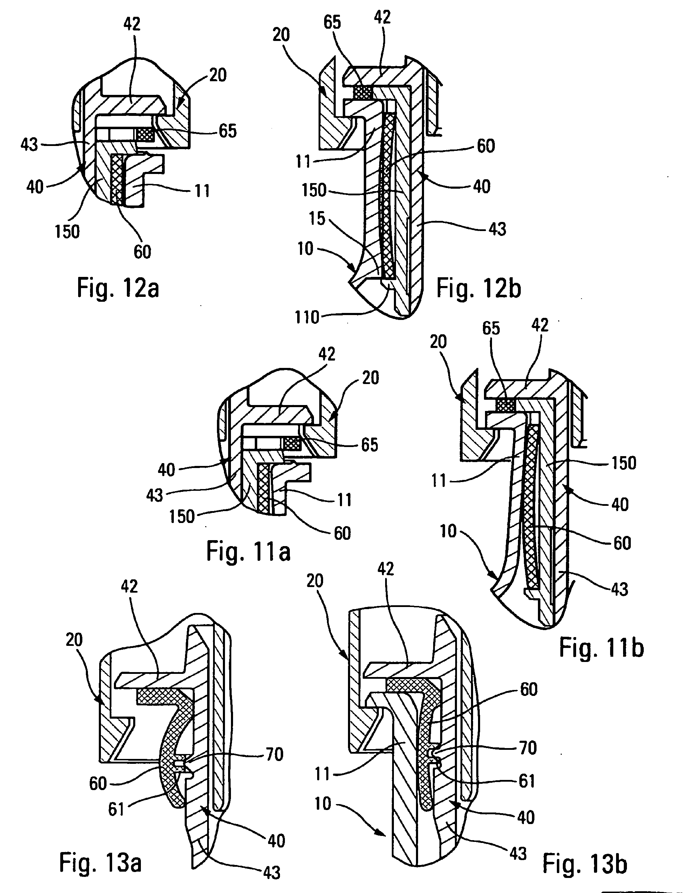

Les figures 12a et 12b représentent une variante de réalisation de celle décrite ci-dessus, dans laquelle l'épaulement inférieur de la douille 150 comporte en outre un profil d'encliquetage 110 qui vient s'encliqueter ou s'harponner sur le profil complémentaire 15 prévu à l'intérieur du col 11 du réservoir.

Les figures 13a et 13b représentent une autre variante de réalisation, dans laquelle il n'y a pas de douille, mais le joint 60 est similaire à celui décrit en référence aux figures 3a et 3b. Cette variante diffère de celle des figures 3a et 3b par l'absence de moyens d'encliquetage à l'extérieur du col 11 du réservoir. La fixation est donc ici également assurée par le serrage radial du joint 60 entre le col 11 du réservoir 10 et le puits axial 43 du piston 40.

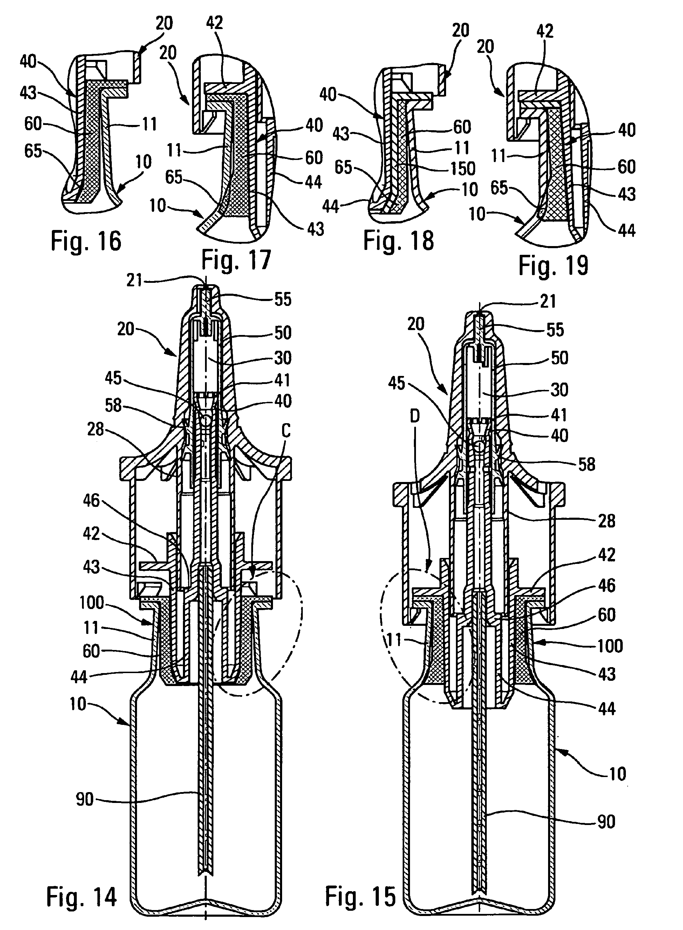

Les figures 14 à 19 représentent encore une autre variante de réalisation. Dans cette variante de réalisation, le joint 60 comporte une projection 65 qui est adaptée à se déformer lors de l'assemblage du piston 40 de telle sorte qu'elle vient se positionner sous le col 11 du réservoir, à l'intérieur de celui-ci, pour assurer une fixation indémontable du dispositif et une étanchéité améliorée. Les figures 16 et 17, qui sont des vues agrandies des détails C et D des figures 14 et 15 montrent clairement comment la projection 65, qui avant assemblage est appliquée contre le piston 40 est déformée lors de l'assemblage par la forme tronconique de l'extrémité inférieure du puits axial 43 du piston qui coopère avec ladite projection 65. Les figures 18 et 19 représentent deux variantes de réalisation respectivement avant et après assemblage, la figure 18 montrant une douille 150 interposée entre le piston 40 et le col 11 du réservoir, le joint 60 étant interposé entre la douille 150 et le col 11. La figure 19 montre elle un joint 60 coopérant avec une partie de joint sous forme de bride radiale réalisée en un matériau différent, ce qui permet d'adapter les exigences d'étanchéité et de flexibilité. Tous les joints décrits en référence aux figures 9 à 19 sont avantageusement réalisés par surmoulage, en particulier lorsqu'ils sont destinés à coopérer avec une douille 150 comme représenté sur les figures 11a à 12b. Dans ce cas, le joint 60 est injecté autour de ladite douille 150, ce qui permet de réaliser ladite extension supérieure 65 qui vient déformer le joint 60 en fin d'assemblage.

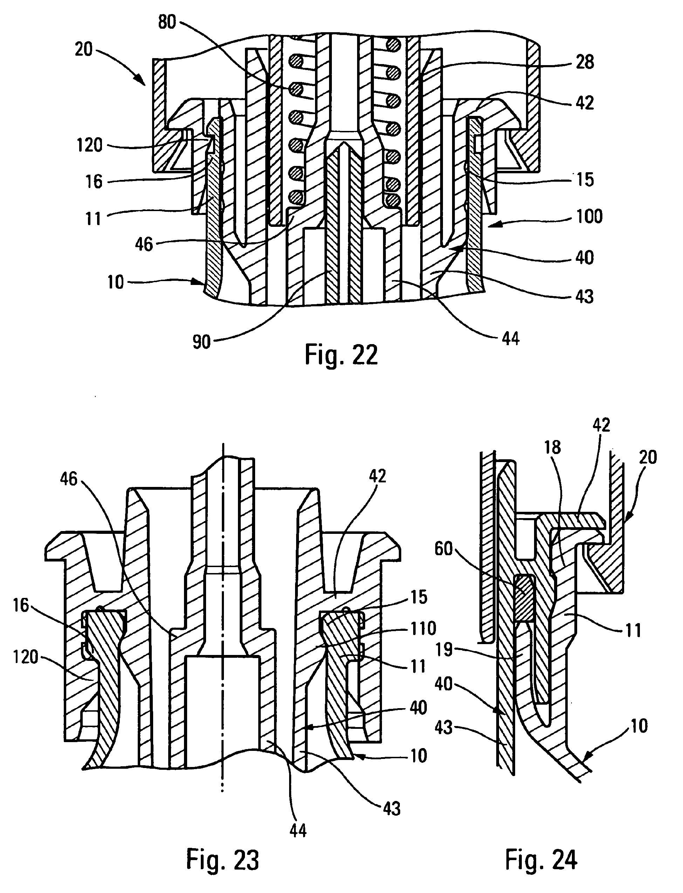

Les figures 20 et 22 à 24 représentent un autre mode de réalisation dans lequel le piston est fixé à la fois à l'intérieur et à l'extérieur du col 11 du réservoir 10. En se référant aux figures 20 et 22, la figure 22 étant une vue agrandie du détail E de la figure 20, on constate que le piston 40 comporte un profil d'encliquetage externe 120 coopérant avec un profil correspondant 16 prévu à l'extérieur du col 11 du réservoir, ledit col de réservoir comportant en outre des bourrelets ou autres profils internes sur sa surface intérieure adaptés à coopérer avec le piston 40 pour assurer l'étanchéité. Bien évidemment, ces profils pourraient également être prévus sur le piston 40 plutôt que dans le col 11 du réservoir. La fixation est donc ici réalisée par encliquetage à l'extérieur et emmanchement à l'intérieur du col du réservoir.

La figure 23 représente une variante de réalisation dans laquelle le piston 40 est fixé sur le col 11 du réservoir par encliquetage à la fois à l'intérieur et à l'extérieur de celui-ci. Dans ce cas, le piston 40 comporte des profils d'encliquetage externe 120 et interne 110 qui coopèrent respectivement avec des profils d'encliquetage correspondants 16 et 15 du col du réservoir 11.

Enfin dans le mode de réalisation de la figure 24, le bord supérieur du col 11 du réservoir 10 comporte deux branches axiales sensiblement parallèles 18 et 19 coopérant chacune avec le piston 40, de préférence avec interposition d'un joint 60. Dans ce cas, l'encliquetage peut se faire à l'intérieur d'une des branches 18 alors que l'autre branche peut supporter ledit joint 60. Bien entendu, des moyens de fixation et d'étanchéité différents pourraient être envisagés, avec une telle configuration complexe du col du réservoir. Il est à noter que ce double encliquetage interne et externe pourrait aussi être utilisé pour fixer un corps de pompe sur un réservoir.

La présente invention permet donc de réaliser un dispositif de distribution de produit fluide qui est simple et facile à assembler, l'assemblage ne présentant aucun risque pour le fonctionnement de la pompe, et notamment n'altérant en aucune manière la surface de glissement du corps de pompe 50 ou du piston 40 lui-même au niveau de sa partie d'étanchéité 41 coulissant dans ledit corps de pompe 50. Au contraire, le fait de fixer le piston 40 directement sur le réservoir, contrairement au dispositif classique dans lequel c'est le corps de pompe qui est fixé au réservoir, permet de réaliser une telle fixation de différentes manières très variées, sûres, simples et fiables. Un autre avantage de cette mise en oeuvre est de permettre de réaliser la chambre de dosage ou de pompe 30 directement en amont de l'orifice de distribution 21, ce qui permet de réaliser l'obturateur 55 et le clapet de sortie de cette chambre de dosage 30 en tant qu'une seule et même pièce. Il n'est donc plus nécessaire de prévoir un clapet de sortie pour la chambre de dosage puis, après un canal d'expulsion s'étendant à travers la tête de distribution, un obturateur séparé dudit clapet au niveau de l'orifice de distribution, comme cela est le cas dans la plupart des dispositifs existants. La présente invention permet donc de réaliser une pompe plus compacte et comportant moins de pièces, et qui est donc moins chère. De même, la présente invention ne comporte qu'un seul ressort 80 qui agit en tant que ressort de rappel de la pompe et pour fermer l'obturateur 55, alors que dans les dispositifs existants, lorsqu'il existe un obturateur placé directement en amont de l'orifice de distribution 21, il y a généralement deux ressorts, l'un pour solliciter l'obturateur vers sa position de fermeture et l'autre pour ramener le piston vers sa position de repos. La présente invention permet donc ici encore d'améliorer et de simplifier les pompes de l'art antérieur.

Bien entendu, l'invention a été décrite en référence à plusieurs variantes de réalisation en référence aux dessins, mais ceux-ci ne sont pas limitatifs, et au contraire l'homme du métier peut y apporter différentes modifications sans sortir du cadre de la présente invention tel que défini par les revendications annexées.

## Revendications

1. Dispositif de distribution de produit fluide, comportant un réservoir (10), une tête de distribution (20) incorporant un orifice de distribution (21), une chambre de dosage (30) et un piston (40) coulissant dans ladite chambre de dosage (30) entre une position de repos et une position de distribution, ledit piston (40) étant monté fixement sur ledit réservoir (10), **caractérisé en ce que** ledit piston est réalisé de manière monobloc avec ledit réservoir (10) et/ou avec des moyens de fixation (100) adaptés à se fixer sur ledit réservoir (10), ledit piston (40) coulissant dans un corps de pompe (50) disposé dans la tête de distribution (20), ladite chambre de dosage (30) étant disposée directement en amont dudit orifice de distribution (21).

2. Dispositif selon la revendication 1, dans lequel ledit piston (40) est fixé sur le réservoir (10) par des moyens de fixation (100), lesdits moyens de fixation (100) étant reliés de manière monobloc audit piston (40).

3. Dispositif selon la revendication 2, dans lequel lesdits moyens de fixation (100) sont des moyens d'encliquetage, d'emmanchement ou de vissage.

4. Dispositif selon la revendication 2 ou 3, dans lequel ledit piston (40) est fixé à l'extérieur d'un col (11) dudit réservoir (10).

5. Dispositif selon la revendication 2 ou 3, dans lequel ledit piston (40) est fixé à l'intérieur d'un col (11) dudit réservoir (10).

6. Dispositif selon la revendication 2 ou 3, dans lequel ledit piston (40) est fixé à l'extérieur et à l'intérieur d'un col (11) dudit réservoir (10).

7. Dispositif selon la revendication 1, dans lequel ledit piston (40) est réalisé de manière monobloc avec ledit réservoir (10).

8. Dispositif selon l'une quelconque des revendications précédentes, dans lequel ledit corps de pompe (50) est solidaire d'un obturateur (55) coulissant dans ladite tête de distribution (20) entre une position de fermeture et une position d'ouverture de l'orifice de distribution (21).

9. Dispositif selon la revendication 8, dans lequel ledit obturateur (55) comporte un second piston (58) coulissant dans un cylindre (28) de ladite tête de distribution (20).

10. Dispositif selon l'une quelconque des revendications précédentes, dans lequel ledit piston (40) comporte un clapet d'entrée (45) de la chambre de pompe (30).

11. Dispositif selon l'une quelconque des revendications précédentes, dans lequel ledit piston (40) est fixé sur le réservoir (10) avec interposition d'un joint (60) entre le piston (40) et le réservoir (10).

12. Dispositif selon la revendication 11, dans lequel ledit joint (60) est déformé lors de la fixation dudit piston (40) sur ledit réservoir (10).

13. Dispositif selon la revendication 11 ou 12, dans lequel le piston (40) comporte des moyens de fixation (100) coopérant avec l'extérieur d'un col (11) du réservoir (10), ledit joint (60) coopérant avec l'intérieur dudit col (11).

14. Dispositif selon la revendication 11 ou 12, dans lequel le piston (40) comporte des moyens de fixation (100) coopérant avec l'intérieur d'un col (11) du réservoir (10).

15. Dispositif selon la revendication 14, dans lequel lesdits moyens de fixation (100) comprennent ledit joint (60) qui se déforme entre le piston (40) et l'intérieur du col (11) du réservoir (10) pour fixer le piston (40) par emmanchement étanche dans ledit col (11).

16. Dispositif selon la revendication 13 ou 15, dans lequel l'une parmi la surface intérieure du col (11) du réservoir (10) et la surface extérieure correspondante du piston (40) comporte un profil d'étanchéité (70) coopérant avec ledit joint (60) à l'état monté, de préférence avec un profil d'étanchéité complémentaire (61) dudit joint (60).

17. Dispositif selon l'une quelconque des revendications 14 à 16, dans lequel ledit joint (60) comporte une projection (65) qui, lors de l'emmanchement du piston (40), est déformée sous le col (11) du réservoir (10), à l'intérieur de celui-ci, pour fixer le piston (40) de manière indémontable.

18. Dispositif selon l'une quelconque des revendications précédentes, dans lequel ledit piston (40) comporte des moyens d'encliquetage (110, 120) coopérant avec la surface intérieure et/ou la surface extérieure d'un col (11) dudit réservoir (10).

19. Dispositif selon la revendication 18, dans lequel lesdits moyens d'encliquetage (110, 120) comportent un profil d'encliquetage (110, 120) adapté à s'encliqueter dans, respectivement autour dudit col (11).

20. Dispositif selon la revendication 19, dans lequel ledit col (11) comporte un profil d'encliquetage complémentaire (15, 16) coopérant avec ledit profil d'encliquetage (110, 120) dudit piston (40).

21. Dispositif selon l'une quelconque des revendications précédentes, dans lequel le piston (40) comporte un filetage (80) coopérant avec un filetage correspondant (85) prévu à l'intérieur ou à l'extérieur d'un col (11) dudit réservoir (10).

22. Dispositif selon la revendication 21, dans lequel au moins un desdits filetages (80, 85) comporte des moyens empêchant le dévissage, tel qu'un filet interrompu.

23. Dispositif selon l'une quelconque des revendications précédentes, dans lequel ledit piston (40) comporte des premiers moyens d'encliquetage (110) coopérant avec l'intérieur d'un col (11) du réservoir (10) et des seconds moyens d'encliquetage (120) coopérant avec l'extérieur dudit col (11).

24. Dispositif selon la revendication 23, dans lequel l'intérieur et/ou l'extérieur dudit col (11) comporte un profil d'encliquetage complémentaire (15, 16).

25. Dispositif selon l'une quelconque des revendications précédentes, dans lequel le bord supérieur d'un col (11) du réservoir (10) comporte deux branches axiales (18, 19) coopérant chacune avec ledit piston (40), de préférence avec interposition d'un joint (60).

26. Dispositif selon la revendication 19 ou 20, dans lequel lesdits moyens d'encliquetage (110, 120) sont formés directement sur le piston.

27. Dispositif selon l'une quelconque des revendications précédentes, dans lequel une douille (150) est interposée entre le piston (40) et un col (11) dudit réservoir (10).

28. Dispositif selon la revendication 27 et l'une des revendications 19 et 20, dans lequel lesdits moyens d'encliquetage (110) sont formés sur ladite douille (150).

29. Dispositif selon la revendication 27 ou 28, dans lequel un joint (60) est interposé entre ladite douille (150) et le col (11) du réservoir (10).

30. Dispositif selon l'une quelconque des revendications précédentes, dans lequel ledit piston (40) est directement emmanché à force à l'intérieur d'un col (11) dudit réservoir (10).

## Claims

1. A fluid dispenser device comprising: a reservoir (10); a dispenser head (20) incorporating a dispenser orifice (21); a metering chamber (30); and a piston (40) that is slidable in said metering chamber (30) between a rest position and a dispensing position, said piston (40) being mounted in stationary manner on said reservoir (10), the device being **characterized in that** said piston is made integrally with said reservoir (10) and/or with fastener means (100) that are adapted to be fastened on said reservoir (10), said piston (40) sliding in a pump body (50) disposed in the dispenser head (20), said metering chamber (30) being provided directly upstream from said dispenser orifice (21).

2. A device according to claim 1, in which said piston (40) is fastened on the reservoir (10) by fastener means (100), said fastener means (100) being connected integrally with said piston (40).

3. A device according to claim 2, in which said fastener means (100) are snap-fastener, force-fit, or screw-fastener means.

4. A device according to claim 2 or claim 3, in which said piston (40) is fastened to the outside of a neck (11) of said reservoir (10).

5. A device according to claim 2 or claim 3, in which said piston (40) is fastened to the inside of a neck (11) of said reservoir (10).

6. A device according to claim 2 or claim 3, in which said piston (40) is fastened both to the outside and to the inside of a neck (11) of said reservoir (10).

7. A device according to claim 1, in which said piston (40) is made integrally with said reservoir (10).

8. A device acceding to any preceding claim, in which said pump body (50) forms part of a closure member (55) that is slidable in said dispenser head (20) between a closed position and an open position of the dispenser orifice (21).

9. A device according to claim 8, in which said closure member (55) includes a second piston (58) that is slidable in a cylinder (28) of said dispenser head (20).

10. A device according to any preceding claim, in which said piston (40) includes an inlet valve (45) of the pump chamber (30).

11. A device according to any preceding claim, in which said piston (40) is fastened on the reservoir (10) with a gasket (60) interposed between the piston (40) and the reservoir (10).

12. A device according to claim 11, in which said gasket (60) is deformed while said piston (40) is being fastened on said reservoir (10).

13. A device according to claim 11 or claim 12, in which the piston (40) includes fastener means (100) co-operating with the outside of a neck (11) of the reservoir (10), said gasket (60) co-operating with the inside of said neck (11).

14. A device according to claim 11 or claim 12, in which the piston (40) includes fastener means (100) co-operating with the inside of a neck (11) of the reservoir (10).

15. A device according to claim 14, in which said fastener means (100) include said gasket (60) that is deformed between the piston (40) and the inside of the neck (11) of the reservoir (10), so as to fasten the piston (40) in said neck (11) by a leaktight force fit.

16. A device according to claim 13 or claim 15, in which one amongst the inside surface of the neck (11) of the reservoir (10) and the corresponding outside surface of the piston (40) includes a sealing profile (70) co-operating with said gasket (60) in the mounted state, preferably with a complementary sealing profile (61) of said gasket (60).

17. A device according to any one of claims 14 to 16, in which said gasket (60) includes a projection (65) that, while the piston (40) is being fitted by force, is itself deformed below the neck (11) of the reservoir (10), inside said reservoir, so as to fasten the piston (40) in permanent manner.

18. A device according to any preceding claim, in which said piston (40) includes snap-fastener means (110, 120) co-operating with the inside surface and/or the outside surface of a neck (11) of said reservoir (10).

19. A device according to claim 18, in which said snap-fastener means (110, 120) comprise a snap-fastener profile (110, 120) that is adapted to be snap-fastened in said neck (11) or around said neck.

20. A device according to claim 19, in which said neck (11) includes a complementary snap-fastener profile (15, 16) co-operating with said snap-fastener profile (110, 120) of said piston (40).

21. A device according to any preceding claim, in which the piston (40) includes screw thread (80) co-operating with a corresponding thread (85) provided on the inside or on the outside a neck (11) of said reservoir (10).

22. A device according to claim 21, in which at least one of said threads (80, 85) includes means for preventing unscrewing, such as a broken portion.

23. A device according to any preceding claim, in which said piston (40) includes first snap-fastener means (110) co-operating with the inside of a neck (11) of the reservoir (10), and second snap-fastener means (120) co-operating with the outside of said neck (11).

24. A device according to claim 23, in which the inside and/or the outside of said neck (11) includes a complementary snap-fastener profile (15, 16).

25. A device according to any preceding claim, in which the top edge of a neck (11) of the reservoir (10) includes two axial branches (18, 19) each co-operating with said piston (40), preferably with a gasket (60) being interposed therebetween.

26. A device according to claim 19 or claim 20, in which said fastener means (110, 120) are formed directly on the piston.

27. A device according to any preceding claim, in which a bushing (150) is interposed between the piston (40) and a neck (11) of said reservoir (10).

28. A device according to claim 27, and claim 19 or claim 20, in which said snap-fastener means (110) are formed on said bushing (150).

29. A device according to claim 27 or claim 28, in which a gasket (60) is interposed between said bushing (150) and the neck (11) of the reservoir (10).

30. A device according to any preceding claim, in which said piston (40) is force fitted directly inside a neck (11) of said reservoir (10).

## Patentansprüche

1. Abgabevorrichtung für ein fluidförmiges Produkt, die einen Behälter (10), einen Abgabekopf (20) mit einer Abgabeöffnung (21), eine Dosierkammer (30) und einen Kolben (40) umfasst, der in der Dosierkammer (30) zwischen einer Ruhelage und einer Abgabestellung gleitet, wobei der Kolben (40) fest auf dem Behälter (10) montiert ist, **dadurch gekennzeichnet, dass** der Kolben mit dem Behälter (10) und/oder mit Befestigungseinrichtungen (100) einstückig ausgebildet ist die geeignet sind, am Behälter (10) befestigt zu werden, wobei der Kolben (40) in einem Pumpenkörper (50) gleitet, der im Abgabekopf (20) angeordnet ist, und wobei die Dosierkammer (30) unmittelbar flussaufwärts von der Abgabeöffnung (21) vorgesehen ist.

2. Vorrichtung nach Anspruch 1, bei welcher der Kolben (40) auf dem Behälter (10) mit Hilfe von Befestigungseinrichtungen (100) befestigt ist, wobei diese Befestigungseinrichtungen (100) einstückig mit dem Kolben (40) verbunden sind.

3. Vorrichtung nach Anspruch 2, bei der die Befestigungseinrichtungen (100) Rasteinrichtungen, Aufpresseinrichtungen oder Schraubeinrichtungen sind.

4. Vorrichtung nach Anspruch 2 oder 3, bei welcher der Kolben (40) außerhalb eines Halses (11) des Behälters (10) befestigt ist.

5. Vorrichtung nach Anspruch 2 oder 3, bei welcher der Kolben (40) innerhalb eines Halses (11) des Behälters (10) befestigt ist.

6. Vorrichtung nach Anspruch 2 oder 3, bei welcher der Kolben (40) an der au-βerhalb und an innerhalb eines Halses (11) des Behälters (10) befestigt ist.

7. Vorrichtung nach Anspruch 1, bei welcher der Kolben (40) mit dem Behälter (10) einstückig ausgebildet ist.

8. Vorrichtung nach einem der vorhergehenden Ansprüche, bei welcher der Pumpenkörper (50) fest mit einem Verschluss (55) verbunden ist, der in dem Abgabekopf (20) zwischen einer Verschlussstellung und einer Öffnungsstellung der Abgabeöffnung (21) gleitet.

9. Vorrichtung nach Anspruch 8, bei welcher der Verschluss (55) einen zweiten Kolben (58) umfasst, der in einem Zylinder (28) des Abgabekopfes (20) gleitet.

10. Vorrichtung nach einem der vorhergehenden Ansprüche, bei welcher der Kolben (40) ein Eintrittsventil (45) für die Pumpenkammer (30) aufweist.

11. Vorrichtung nach einem der vorhergehenden Ansprüche, bei welcher der Kolben (40) auf dem Behälter (10) unter Zwischenfügung einer Dichtung (60) zwischen dem Kolben (40) und dem Behälter (10) befestigt ist.

12. Vorrichtung nach Anspruch 11, bei welcher die Dichtung (60) während der Befestigung des Kolbens (40) auf dem Behälter (10) verformt wird.

13. Vorrichtung nach Anspruch 11 oder 12, bei welcher der Kolben (40) Befestigungseinrichtungen (100) aufweist, die mit dem Außenbereich eines Halses (11) des Behälters (10) zusammenwirken, wobei die Dichtung (60) mit dem Inneren des Halses (11) zusammenwirkt.

14. Vorrichtung nach Anspruch 11 oder 12, bei welcher der Kolben (40) Befestigungseinrichtungen (100) umfasst, die mit dem Inneren eines Halses (11) des Behälters (10) zusammenwirken.

15. Vorrichtung nach Anspruch 14, bei welcher die Befestigungseinrichtungen (100) die Dichtung (60) umfassen, die sich zwischen dem Kolben (40) und dem Inneren des Halses (11) des Behälters (10) verformt, um den Kolben (40) durch dichtes Einpressen in den Hals (11) zu befestigen.

16. Vorrichtung nach Anspruch 13 oder 15, bei welcher entweder die innere Oberfläche des Halses (11) des Behälters (10) oder die entsprechende äußere Oberfläche des Kolbens (40) ein Dichtprofil (70) aufweist, das im montierten Zustand mit der Dichtung (60), vorzugsweise mit einem komplementären Dichtprofil (61). der Dichtung (60) zusammenwirkt

17. Vorrichtung nach einem der Ansprüche 14 bis 16, bei welcher die Dichtung (60) einen Vorsprung (65) umfasst, der während des Einpressens des Kolbens (40) unter dem Hals (11) des Behälters (10) in dessen Inneren verformt wird, um den Kolben (40) in nicht-lösbarer Weise zu befestigen.

18. Vorrichtung nach einem der vorhergehenden Ansprüche, bei welcher der Kolben (40) Rasteinrichtungen (110,120) umfasst, die mit der inneren und/oder äußeren Oberfläche eines Halses (11) des Behälters (10) zusammenwirken.

19. Vorrichtung nach Anspruch 18, bei welcher die Rasteinrichtungen (110,120) ein Rastprofil (110,120) umfassen, das geeignet ist, entweder im Hals (11) oder um diesen Hals herum einzurasten.

20. Vorrichtung nach Anspruch 19, bei welcher der Hals (11) ein komplementäres Rastprofil (15,16) aufweist, das mit dem Rastprofil (110,120) des Kolbens (40) zusammenwirkt.

21. Vorrichtung nach einem der vorhergehenden Ansprüche, bei welcher der Kolben (40) ein Gewinde (80) aufweist, das mit einem entsprechenden Gewinde (85) zusammenwirkt, das innerhalb oder außerhalb eines Halses (11) des Behälters (10) vorgesehen ist.

22. Vorrichtung nach Anspruch 21, bei welcher wenigstens eines der Gewinde (80,85) Einrichtungen umfasst, die ein Abschrauben verhindern, wie z.B. ein unterbrochenes Gewinde.

23. Vorrichtung nach einem der vorhergehenden Ansprüche, bei welcher der Kolben (40) erste Rasteinrichtungen (110), die mit dem Inneren eines Halses (11) des Behälters (10) zusammenwirken, und zweite Rasteinrichtungen (120) umfasst, die mit der Außenseite des Halses (11) zusammenwirken.

24. Vorrichtung nach Anspruch 23, bei der das Innere und/oder das Äußere des Halses (11) ein komplementäres Rastprofil (15,16) aufweist.

25. Vorrichtung nach einem der vorhergehenden Ansprüche, bei welcher der obere Rand eines Halses (11) des Behälters (10) zwei axiale Verzweigungen (18,19) aufweist, von denen jede mit dem Kolben (40) vorzugsweise unter Zwischenfügung einer Dichtung (60) zusammenwirkt.

26. Vorrichtung nach Anspruch 19 oder 20, bei welcher die Rasteinrichtungen (110,120) direkt an den Kolben ausgebildet sind.

27. Vorrichtung nach einem der vorhergehenden Ansprüche, bei welcher eine Tülle (150) zwischen dem Kolben (40) und einem Hals (11) des Behälters (10) zwischengefügt ist.

28. Vorrichtung nach Anspruch 27 und einem der Ansprüche 19 und 20, bei welcher die Rasteinrichtungen (110) an der Tülle (150) ausgebildet sind.

29. Vorrichtung nach Anspruch 27 oder 28, bei der eine Dichtung (60) zwischen die Tülle (150) und den Hals (11) des Behälters (10) eingefügt ist.

30. Vorrichtung nach einem der vorhergehenden Ansprüche, bei welcher der Kolben (40) direkt in das Innere eines Halses (11) des Behälters (10) mit Kraft eingepresst ist.
